# EUROPÄISCHE PATENTANMELDUNG

(11) **EP 2 008 577 A1**
(43) Veröffentlichungstag der Anmeldung: **31.12.2008**
(21) Anmeldenummer: 07012663.6
(22) Anmeldetag: 28.06.2007
(51) Int. Cl.: A61B 5/00, A61M 5/142

(54) **Anordnung mit Infusionspumpe und Messgerät**

(71) Anmelder: F.HOFFMANN-LA ROCHE AG, 4070 Basel (CH); Roche Diagnostics GmbH, 68305 Mannheim (DE)
(72) Erfinder: Haueter, Ulrich, 3506 Grosshöchstetten (CH); Kühni, Florian, 3400 Burgdorf (CH)
(74) Vertreter: Schalch, Rainer

(57) **Zusammenfassung**

Bei einer Insulinpumpe (1) ist mindestens ein Alarmgeber (21) durch ein Signal (17) aus einem Blutzuckermessgerät (11) deaktivierbar. Dadurch können Alarme an der Pumpe (1) unterdrückt werden, die auf Grund von Messungen unnötig sind. Dies erhöht den Bedienungskomfort der Anordnung aus Pumpe und Messgerät.

## Beschreibung

Die Erfindung betrifft eine Anordnung umfassend mindestens eine Infusionspumpe und mindestens ein Messgerät zur Messung eines Körperwerts des die Infusion erhaltenden Patienten, wobei die Infusionspumpe mindestens einen Alarmgeber aufweist. Weiter betrifft die Erfindung eine Insulinpumpe, ein Blutzuckermessgerät und ein Verfahren zur Verabreichung einer Insulininfusion.

Infusionspumpen, insbesondere am Körper tragbare Insulinpumpen, sind bekannt. Solche Insulinpumpen liefern Insulin gemäss der eingestellten Basalrate und geben zusätzlich benötigtes Insulin als Bolus ab, was hier nicht weiter erläutert wird. Auch Messgeräte für Körperwerte sind in verschiedener Ausführung bekannt, insbesondere als Blutzuckermessgeräte, z.B. als mit Messstreifen arbeitende Blutzuckermessgeräte. Bei Insulinpumpen ist es bekannt, dass der Benutzer Alarme eines Alarmgebers programmieren kann, die als Erinnerung dienen, und dass Alarme eines Alarmgebers auch von der Pumpe selber als auf Ereignisse folgende Signale abgegeben werden, um den Benutzer ebenfalls auf notwendige oder ratsame Handlungen hinzuweisen. Als Beispiel für eine Infusionspumpe solcher Art kann auf US-B-6,852,104 verwiesen werden.

Der Erfindung liegt die Aufgabe zu Grunde eine Anordnung aus Infusionspumpe und Messgerät, insbesondere Insulinpumpe und Blutzuckermessgerät zu verbessern.

Diese Aufgabe wird bei einer Anordnung der eingangs genannten Art dadurch gelöst, dass der Alarm des Alarmgebers durch ein von der Infusionspumpe drahtlos oder drahtgebunden empfangbares Signal des Messgerätes deaktivierbar ist.

Dadurch, dass ein Alarmgeber die Infusionspumpe durch ein Signal des Messgerätes deaktivierbar ist, ergibt sich eine Verminderung von unnötigen Alarmmeldungen. Zum Beispiel derartige Alarme, welche den Benutzer bisher an Aktionen erinnert haben, die dieser aber schon von sich aus durchgeführt hat oder die situationsbedingt nicht notwendig sind, können unterdrückt werden.

Der Erfindung liegt ferner die Aufgabe zu Grunde eine Insulinpumpe zu verbessern bzw. ein Blutzuckermessgerät zu verbessern.

Dies wird mit den entsprechenden auf die Insulinpumpe gerichteten Patentansprüchen bzw. mit den entsprechenden auf das Blutzuckermessgerät gerichteten Patentansprüchen erreicht.

Der Erfindung liegt ferner die Aufgabe zu Grunde ein Verfahren zur Verabreichung einer Insulininfusion zu verbessern, was mit den entsprechenden Verfahrenspatentansprüchen erreicht wird.

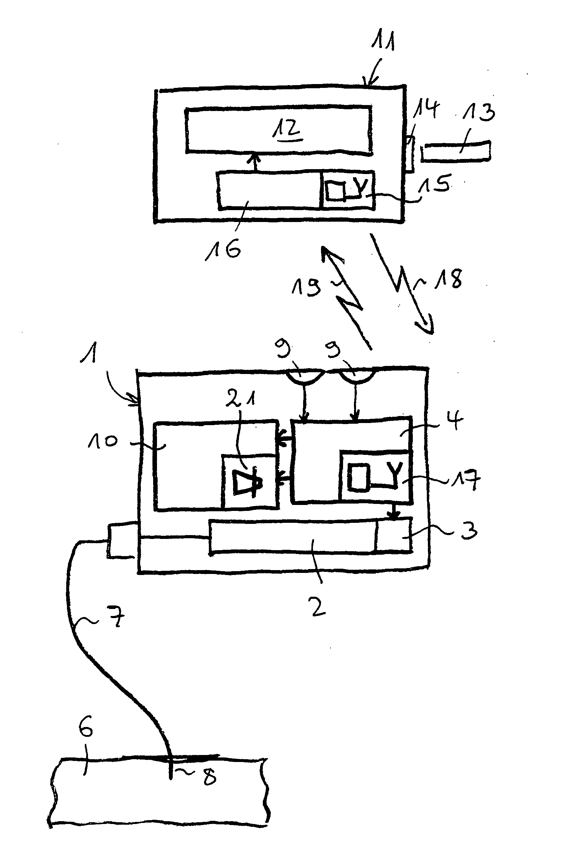

Im Folgenden werden Ausführungsbeispiele der Erfindung anhand der Figur näher erläutert.

Die Figur zeigt schematisch eine Anordnung aus einer Insulinpumpe und einem Blutzuckermessgerät.

Die Insulinpumpe 1, welche hier als bevorzugtes Beispiel für eine Infusionspumpe gezeigt ist, weist einen Insulinvorratsbehälter 2 auf. Die Steuerung 4 der Pumpe bewirkt über einen Antrieb 3 und einen nicht dargestellten Kolben eine gesteuerte und genau dosierte Abgabe von Insulin an einen Patienten 6. Dies erfolgt über eine Infusionsleitung 7 und eine im Körper des Patienten befindliche Kanüle 8. Solche Insulinpumpen sind bekannt und handelsüblich, so dass deren Aufbau hier dem Fachmann nicht näher erläutert werden muss. Die Infusionspumpe bzw. Insulinpumpe weist ferner Bedienungsorgane 9, z.B. Druckknöpfe zur Befehlseingabe und eine Anzeige (Display) 10 auf, welche von der Steuerung der Pumpe betrieben wird. Nicht dargestellt ist die Stromversorgung der Insulinpumpe, die bei einer am Körper tragbaren Pumpe eine Batteriestromversorgung ist. Weiter ist es bekannt, dass die Infusionspumpe mindestens einen Alarmgeber bzw. Signalgeber 21 aufweist. Dieser kann ein hörbares und/oder fühlbares Alarmsignal für den Infusionspumpenträger bewirken, also ein Tonsignal und/oder ein Vibrationssignal; dieses Alarmsignal kann verschiedene Funktionen haben und ist in der Regel entsprechend verschieden gestaltet (z.B. mittels Tonfolge und/oder Tonhöhe), so dass der Benutzer die Funktion des Alarmsignals erkennen kann und es von anderen Alarmen unterscheiden kann. Es muss sich insbesondere nicht um ein Gefahr bedeutendes Alarmsignal, also einen Alarm im engeren Sinne handeln. Im Gegenteil werden Gefahr bedeutende Alarme, also z.B. bei einer Okklusion der Infusionsleitung in der Regel nicht gemäss der vorliegenden Erfindung deaktivierbar sein. In der vorliegenden Anmeldung wird aber der Einfachheit immer von Alarm und Alarmgeber gesprochen, auch wenn es sich um ein gewöhnliches Signal bzw. um einen Signalgeber handelt. Es können in der Infusionspumpe verschiedene Alarmgeber vorhanden sein, wovon mindestens ein Alarmgeber gemäss der Erfindung ausgestaltet ist. Der Alarmgeber 21 wird von der Steuerung 4 oder von einer separaten Alarmgeberschaltung betrieben. Ein Alarm kann durch ein Ereignis in der Pumpe ausgelöst werden (direkt oder zeitverzögert), wie z.B. durch den Austausch des Vorratsbehälters 2, oder der Alarm kann durch den Benutzer auf eine bestimmte Zeit eingestellt oder nach einer bestimmten Zeitdauer erfolgend programmiert werden. In diesem Fall übernimmt der Alarm des Alarmgebers eine Erinnerungsfunktion für den Benutzer, was nachfolgend noch erläutert wird. Die Einstellung oder Programmierung des Alarms erfolgt dabei über die Bedienungsorgane 9 und mit Hilfe der Anzeigeeinrichtung 10. Die Einstellung von ereignisbedingten Alarmen erfolgt über die Steuerung mit oder ohne zutun des Benützers.

Das Messgerät 11, im gezeigten Beispiel ein Blutzuckermessgerät, besitzt auf bekannte Weise eine Möglichkeit zur Messung mindestens eines Körperwertes des Patienten, im Falle des Blutzuckermessgerätes des Blutzuckerwertes. Dazu kann auf bekannte Weise Blut auf einem Teststreifen 13 durch eine Einschuböffnung 14 in das Gerät 11 eingeführt werden, wo auf ebenfalls bekannte Weise die Messung erfolgt. Dies muss hier nicht weiter erläutert werden, da diese Messverfahren sowie alternativ anwendbare Messverfahren dem Fachmann vertraut sind. In der Regel weist das Gerät ferner eine Anzeige 12 auf. Eine Steuerschaltung 16 im Gerät 11 steuert die Messung und die Anzeige 12.

Gemäss der Erfindung sind nun Infusionspumpe 1 und Messgerät 11 miteinander zum Signalaustausch ausgebildet. Es kann sich um eine drahtgebundene Verbindung handeln oder bevorzugterweise um eine drahtlose Verbindung. In der Figur ist eine Funkverbindung durch Module 15 und 17 im Gerät 11 bzw. in der Infusionspumpe 1 dargestellt sowie durch einen Pfeil 18 als Verbindung vom Gerät 11 zur Infusionspumpe 1 (unidirektionale Signalverbindung vom Gerät 11 zur Pumpe 1). Zusätzlich kann eine Signalverbindung auch von der Infusionspumpe 1 zum Gerät 11 vorgesehen sein, vorzugsweise ebenfalls über die Module 17 und 15, was durch den Pfeil 19 angedeutet ist. Diese Module können z.B. eine Funkverbindung gemäss dem Bluetooth^{®} Standard zwischen Gerät 11 und Pumpe 1 herstellen. Die Pumpe 1 und das Messgerät 11 sind vorzugsweise so ausgebildet, dass eine gegenseitige Identifikation und Verifikation beim Signalaustausch stattfindet, so dass die Pumpe und das Messgerät Signale von anderen Geräten nicht beachten; solches ist dem Fachmann bekannt und wird hier nicht weiter erläutert.

Gemäss der Erfindung ist nun die Infusionspumpe bzw. die tragbare Insulinpumpe 1 so ausgeführt, dass der Alarm des Alarmgebers 21 durch ein Signal des Messgerätes 11 deaktivierbar ist. Dies kann auf verschiedenen Weise erfolgen. So kann das Signal 18 ein spezifisches, im Gerät 11 bereitgestelltes Signal sein, welches als Information lediglich beinhaltet, dass mindestens ein vorbestimmter Alarm des Alarmgebers 21 in der Infusionspumpe deaktiviert werden soll, bzw. dass der Alarm eines vorbestimmten Alarmgebers unter mehreren Alarmgebern deaktiviert werden soll. Dieses Signal wird nach Empfang durch das Modul 17 von der Steuerung 4 der Pumpe 1 ausgewertet und ein bereits eingestellter Alarm wird gelöscht oder die Steuerung unterdrückt den nächsten durch ein Ereignis ausgelösten Alarm auf Grund des empfangenen Signals vom Gerät 11. Dabei werden nur solche Alarme unterdrückt, die entsprechend als unterdrückbar in der Steuerung gekennzeichnet sind. Das vom Modul 17 empfangene Signal kann aber auch direkt, ohne Einfluss der Steuerung auf den Alarmgeber einwirken bzw. diesen zeitweise deaktivieren. Unter Deaktivierung bzw. Unterdrückung eines Alarms wird dabei verstanden, dass entweder ein bereits gesetzter Alarm nicht ausgelöst oder gelöscht wird, oder auch, dass ein Alarm von der Pumpe 1 gar nicht gesetzt wird, wenn zuvor das von dem Messgerät abgegebene Signal empfangen worden und von der Steuerung 4 entsprechend ausgewertet worden ist. Ferner wird darunter auch verstanden, dass ein gesetzter Alarm zeitlich verschoben wird.

Die Infusionspumpe kann auch mehrere Alarmgeber oder mehrere Schaltungen zum Betrieb eines gemeinsamen Alarmgebers aufweisen, von denen mindestens einer bzw. eine durch das Signal deaktivierbar ist und mindestens ein weiterer Alarmgeber bzw. eine weitere Schaltung durch das Signal nicht beeinflussbar ist. Verschiedene Alarmtypen unterscheiden sich dann dadurch, ob sie als deaktivierbare Alarme über den einen Alarmgeber oder als nicht deaktivierbare Alarme über den anderen Alarmgeber abgegeben bzw. über die eine oder andere Alarmschaltung laufen.

Das Signal aus dem Gerät 11 kann aber auch ein Signal sein, welches weitere Informationen enthält, so z.B. einen Messwert, und nur als Zusatz eine Angabe zur Deaktivierung des Alarms in der geschilderten Weise. Weiter kann das Signal aus dem Gerät 11 nur ein Signal mit Informationen ohne spezifische Alarminformation sein und die Infusionspumpe ist so ausgestaltet, dass sie aus dem Messsignal bzw. aus dem Vergleich desselben mit vorgegebenen Messwerten oder einem Messwertbereich entscheidet, ob der Alarm deaktiviert werden soll oder nicht. Dies kann durch die Steuerung 4 der Infusionspumpe geschehen.

Bei einer bevorzugten Ausgestaltung kann die Infusionspumpe 1 derart ausgebildet sein, dass ein Alarm eines Alarmgebers durch das Signal vom Messgerät 11 aktivierbar ist. In diesem Fall wird also durch das vom Messgerät ausgesandte und von der Pumpe 1 empfangene Signal ein Alarmgeber aktiviert, entweder direkt oder über die Steuerung 4. Dies kann zur sofortigen oder zur zeitverzögerten Abgabe eines Alarms führen.

Bei einer besonderen Ausführungsform der Erfindung kann auch ein Signal 19 von der Infusionspumpe 1 an das Gerät 11 abgegeben werden und kann dort einen nicht dargestellten Alarmgeber des Gerätes 11 auf die selbe Weise beeinflussen, wie dies für den Alarmgeber der Infusionspumpe 1 erläutert worden ist. Insbesondere kann also der Alarmgeber im Messgerät deaktiviert und/oder gesetzt werden.

Im Folgenden werden Beispiele gegeben, wie die neue Möglichkeit zur Deaktivierung oder zur Aktivierung eines Alarms den Gebrauchswert der Insulinpumpe oder des Blutzuckermessgerätes verbessert:
1. Bei bekannten Insulinpumpen wird ein Alarm als Erinnerungsalarm sofort oder nach einer vorbestimmten Zeit erzeugt, nachdem bei der Insulinpumpe 1 Ereignisse ausgelöst worden sind, z.B. der Wechsel des Insulinvorratsbehälters 2 oder die Abgabe eines Mahlzeitenbolus. Diese Erinnerungsalarme fordern den Pumpenträger dazu auf, eine Blutzuckermessung auszuführen. Gemäss der vorliegenden Erfindung kann ein solcher Erinnerungsalarm durch das Signal des Blutzuckermessgerätes deaktiviert werden. Dies erfolgt vorzugsweise derart, dass die Infusionspumpe auf Grund des Signals des Messgerätes den Erinnerungsalarm deaktiviert, falls das Signal innert einer vorbestimmten, z.B. einer einstellbaren oder fest programmierten Zeitspanne, vor dem Alarmzeitpunkt vorliegt oder vorgelegen hat. So kann z.B. der Boluserinnerungsalarm deaktiviert werden, bzw. gar nicht in der Pumpe gesetzt werden oder zwar gesetzt werden, aber nicht ausgelöst werden, wenn z.B. innert einer Stunde vor dem Alarmzeitpunkt bereits ein Signal des Blutzuckermessgerätes vorgelegen hat. Dies bedeutet, dass innert der letzten Stunde vor dem Alarmzeitpunkt bereits eine Blutzuckermessung durch das Gerät 11 ausgeführt worden ist, so dass die Erinnerung an eine Messung derzeit nicht notwendig ist. Es ist auch möglich, den Erinnerungsalarm zu deaktivieren, indem er zeitlich verschoben wird, so dass er, abhängig von dem Signal, anstelle zu dem vorgesehenen Zeitpunkt zu einem vorbestimmt späteren Zeitpunkt erfolgt.
2. Bei einem weiteren Beispiel wird bei bekannten Insulinpumpen durch ein Ereignis, z.B. bei einer Bolusverabreichung, ein Timer aktiviert, bei dessen Ablaufen ein Alarm gegeben wird, z.B. 3 Stunden nach der Bolusverabreichung. Wird nun vor dem Ablauf dieser Zeit eine Messung durch das Gerät 11 ausgeführt und damit ein Signal 18 für die Pumpe 1 erzeugt, so setzt diese den Timer zurück, so dass der Alarm nicht erfolgt bzw. deaktiviert ist.
3. Bei einem weiteren Beispiel sind bekannte Insulinpumpen mit einem Alarm ausgestattet, der ausgelöst wird, wenn der Pumpenträger während einer vorbestimmten Zeit, z.B. 12 Stunden, keine Bedienoperationen an der Pumpe vorgenommen hat. Auch dieser Alarm kann deaktiviert werden, wenn das Gerät 11 mittels eines Signals oder in diesem Fall vorzugsweise mittels mehreren, zeitlich beabstandeten Signalen, angezeigt hat, dass der Blutzucker in dieser Zeit genügend oft gemessen worden ist. Dieser Alarm wird also vorzugsweise nur deaktiviert, wenn innerhalb der vorbestimmten Zeit mehrere Signale 18 des Gerätes 11 bei der Pumpe eingetroffen sind.
4. Ein weiteres Beispiel wird durch die Ausführung möglich, bei der ein Signal des Gerätes 11 einen Alarm setzen kann. Liegen innert einer vorbestimmten Zeitspanne mehrere Signale 18 in der Form von Messwerten vor, die von der Pumpe als einen vorgegebenen Blutzuckermesswert unterschreitend erkannt werden, so wird ein Alarm ausgegeben, der den Pumpenträger auffordert, die temporäre Reduktion der Basalrate auszulösen. Bei einer bevorzugten Ausführungsform kann die Pumpe 1 so ausgeführt sein, dass bei einer fehlenden Reaktion des Pumpenträgers auf diesen Alarm innert einer vorgegebenen Zeit diese Funktion der Pumpe selbsttätig ausgelöst wird.
5. Ein weiteres Beispiel betrifft die Deaktivierung eines Mahlzeiten Bolusalarms. Ein solcher erinnert den Pumpenträger einen Mahlzeitenbolus innert eines vorbestimmten Zeitintervalls auszulösen. Auch ein solcher zu vorbestimmter Zeit gesetzter Alarm kann deaktiviert werden, wenn ein Signal 18 des Messgerätes vorliegt, vorzugsweise ein Signal 18 enthaltend einen Messwert, der von der Pumpe als in einem vorbestimmten Wertebereich liegend erkannt wird.
6. Ein weiteres Beispiel umfasst die Alarmsetzung bzw. Auslösung bei dem Gerät 11 auf Grund eines Signals 19 von der Pumpe 1 zum Gerät 11. So kann der Pumpenträger durch einen Alarm des Blutzuckermessgerätes darauf aufmerksam gemacht werden, dass ein Bolusbefehl an der Pumpe von dieser ausgeführt wird, ohne dass zuvor mit dem Gerät 11 eine Messung ausgeführt worden ist. Die Pumpe prüft also bei der Ausführung eines Bolusbefehls, ob innert einer vorbestimmten vorherigen Zeitspanne ein Signal 18 vom Gerät 11 eingegangen ist, was bedeutet, dass eine Messung durchgeführt worden ist. Ist dies nicht der Fall, so löst die Pumpe 1 über ein Signal 19 einen Alarm beim Gerät 11 aus, der den Pumpenträger an die Messung erinnert. Weiter kann generell oder situativ ein Alarm am Gerät 11 nach einer Bolusauslösung bei der Pumpe und Ablauf einer vorbestimmten Zeitspanne gegeben werden, z.B. 2 Stunden nach der Bolusauslösung.
7. Bei einem weiteren Beispiel können Timer bzw. Alarme, die auf dem Gerät 11 gesetzt sind, um an Messungen zu erinnern, über ein Signal 18 auch an die Pumpe 1 übertragen und dort gesetzt werden. Da die Pumpe 1 immer beim Benutzer ist, wird dieser auch an die notwendigen Messungen erinnert, wenn er das Blutzuckermessgerät nicht in Alarmhörweite hat. Diese Alarmsignale werden vorzugsweise so gestaltet, bzw. bestehen aus solchen Ton- und/oder Vibrationsabfolgen, die der Pumpenbenützer als Aufforderung zur Blutzuckermessung versteht.

## Patentansprüche

1. Anordnung umfassend mindestens eine Infusionspumpe (1) und mindestens ein Messgerät (11) zur Messung eines Körperwerts des die Infusion erhaltenden Patienten, wobei die Infusionspumpe mindestens einen Alarmgeber (21) aufweist, **dadurch gekennzeichnet, dass** der Alarm des Alarmgebers durch ein Signal (18) des Messgerätes (11), das von der Infusionspumpe (1) drahtlos oder drahtgebunden empfangbar ist, deaktivierbar ist.

2. Anordnung nach Anspruch 1, **dadurch gekennzeichnet, dass** der Alarmgeber durch ein Signal des Messgerätes aktivierbar ist.

3. Anordnung nach Anspruch 1 oder 2, **dadurch gekennzeichnet, dass** durch die Infusionspumpe (1) drahtlos oder drahtgebunden ein vom Messgerät (11) empfangbares Signal (19) abgebbar ist, welches einen Alarmgeber des Messgerätes aktiviert und/oder deaktiviert.

4. Anordnung nach einem der Ansprüche 1 bis 3, **dadurch gekennzeichnet, dass** die Infusionspumpe eine am Körper tragbare Insulinpumpe ist und dass das Messgerät ein Blutzuckermessgerät ist.

5. Anordnung nach einem der Ansprüche 1 bis 3, **dadurch gekennzeichnet, dass** die Infusionspumpe eine im Körper des Patienten implantierbare Infusionspumpe und insbesondere eine Insulinpumpe ist.

6. Anordnung nach einem der Ansprüche 1 bis 5, **dadurch gekennzeichnet, dass** das Signal ein Signal ist, dass als Information nur die Deaktivierung des Alarms enthält und/oder, dass das Signal mindestens einen vom Messgerätes ermittelten Messwert, insbesondere einen Blutzuckermesswert, enthält.

7. Anordnung nach einem der Ansprüche 1 bis 6, **dadurch gekennzeichnet, dass** ein Alarm deaktivierbar ist, wenn ein Signal (18) des Messgerätes enthaltend einen Messwert vorliegt, und die Pumpe (1) derart ausgestaltet ist, dass durch sie das Signal als in einem vorbestimmten Wertebereich liegend erkennbar ist.

8. Anordnung nach einem der Ansprüche 1 bis 7, **dadurch gekennzeichnet, dass** durch die Pumpe ein deaktivierbarer Alarm als Erinnerungsalarm sofort oder nach einer vorbestimmten Zeit erzeugbar ist, nachdem bei der Pumpe (1) ein Ereignis ausgelöst worden ist, insbesondere der Wechsel eines Vorratsbehälters (2) oder die Abgabe eines Mahlzeitenbolus, wobei das Signal des Messgerätes den Alarm vorzugsweise derart deaktiviert, dass die Infusionspumpe auf Grund des Signals des Messgerätes den Erinnerungsalarm deaktiviert, falls das Signal innert einer vorbestimmten, insbesondere einer einstellbaren oder fest programmierten, Zeitspanne, vor dem Alarmzeitpunkt vorliegt oder vorgelegen hat.

9. Anordnung nach einem der Ansprüche 1 bis 8, **dadurch gekennzeichnet, dass** die Pumpe mit einem Alarm ausgestattet ist, der ausgelöst wird, wenn der Pumpenträger während einer vorbestimmten Zeit keine Bedienoperationen an der Pumpe vorgenommen hat, wobei dieser Alarm deaktivierbar ist, wenn das Messgerät (11) mittels eines Signals, oder vorzugsweise mittels mehreren Signalen, angezeigt hat, dass der Körperwert, vorzugsweise der Blutzucker, in dieser Zeit genügend oft gemessen worden ist.

10. Anordnung nach einem der Ansprüche 1 bis 9, **dadurch gekennzeichnet, dass** der Erinnerungsalarm durch das Signal deaktivierbar ist, indem er zeitlich verschiebbar ist, so dass er, abhängig von dem Signal, anstelle zum vorgesehenen Zeitpunkt zu einem vorbestimmt späteren Zeitpunkt erfolgt.

11. Anordnung nach einem der Ansprüche 2 bis 10, **dadurch gekennzeichnet, dass** durch ein Signal des Messgerätes (11) ein Alarm in der Pumpe (1) setzbar ist, falls innert einer vorbestimmten Zeitspanne mehrere Signale (18) vom Messgerät (11) in der Form von Messwerten vorliegen, die von der Pumpe als einen vorgegebenen Messwert, insbesondere Blutzuckermesswert, unterschreitend erkennbar sind

12. Anordnung nach Anspruch 11, **dadurch gekennzeichnet**, die Pumpe (1) so ausgeführt ist, dass bei einer fehlenden Reaktion des Pumpenträgers auf den Alarm innert einer vorgegebenen Zeit eine Funktion der Pumpe selbsttätig auslösbar ist.

13. Anordnung nach einem der Ansprüche 3 bis 12, **dadurch gekennzeichnet, dass** die Alarmsetzung bzw. Auslösung bei dem Messgerät (11) mittels des Signals (19) der Pumpe (1) erfolgt, falls ein Bolusbefehl an der Pumpe von dieser ausgeführt worden ist, ohne dass zuvor innert einer vorbestimmten Zeitspanne mit dem Messgerät (11) eine Messung ausgeführt worden ist.

14. Anordnung nach einem der Ansprüche 1 bis 13, **dadurch gekennzeichnet, dass** Timer, die auf dem Messgerät (11) gesetzt sind über das Signal (18) an die Pumpe (1) übertragbar und dort setzbar sind.

15. Infusionspumpe mit mindestens einem Alarmgeber (21), **dadurch gekennzeichnet, dass** der Alarmgeber durch ein von der Infusionspumpe drahtlos oder drahtgebunden empfangbares Signal eines von der Pumpe getrennten Gerätes (11) deaktivierbar ist.

16. Infusionspumpe nach Anspruch 15, **dadurch gekennzeichnet, dass** der Alarmgeber durch ein Signal des Gerätes aktivierbar ist.

17. Infusionspumpe nach Anspruch 15 oder 16, **dadurch gekennzeichnet, dass** das Signal einen Messwert enthält, und dass der Alarmgeber abhängig von einer Auswertung des Messwertes durch die Pumpe, ggf. durch die Auswertung mehrerer zeitlich hintereinander empfangener Signale mit Messwerten, insbesondere durch einen Vergleich des Messwertes oder der Messwerte mit einem vorgegebenen Messwert oder Messwertebereich, deaktiviert oder nicht deaktiviert wird.

18. Infusionspumpe nach einem der Ansprüche 15 bis 18, **dadurch gekennzeichnet, dass** durch die Infusionspumpe (1) drahtlos oder drahtgebunden ein Signal (19) abgebbar ist, welches zum Empfang durch ein Messgerät (11) bestimmt ist und zur Aktivierung und/oder Deaktivierung mindestens eines Alarmgebers des Messgerätes bestimmt ist.

19. Infusionspumpe nach einem der Ansprüche 15 bis 18, **dadurch gekennzeichnet, dass** die Infusionspumpe eine am Körper tragbare Insulinpumpe ist.

20. Infusionspumpe nach einem der Ansprüche 15 bis 18, **dadurch gekennzeichnet, dass** die Infusionspumpe eine im Körper eines Patienten implantierbare Infusionspumpe und insbesondere eine Insulinpumpe ist.

21. Infusionspumpe nach einem der Ansprüche 15 bis 19 sowie entsprechend der Infusionspumpe gemäss der Anordnung nach einem der Ansprüche 6 bis 14.

22. Blutzuckermessgerät, **dadurch gekennzeichnet, dass** es zur drahtgebundenen oder drahtlosen Abgabe eines Signals (18) ausgestaltet ist, welches zur Deaktivierung und/oder Aktivierung eines Alarmgebers (21) in einer Insulinpumpe ausgestaltet ist.

23. Blutzuckermessgerät nach Anspruch 22, **dadurch gekennzeichnet, dass** es zum drahtgebundenen oder drahtlosen Empfang eines Signals (19) ausgestaltet ist, durch welches ein Alarmgeber im Messgerät aktivierbar und/oder deaktivierbar ist.

24. Blutzuckermessgerät nach einem der Ansprüche 22 oder 23 und gemäss einer Anordnung nach einem der Ansprüche 1 bis 14.

25. Verfahren zur Verabreichung einer Insulininfusion, mit einer am Körper tragbaren Insulinpumpe, umfassend neben der Insulinpumpe (1) mindestens ein Blutzuckermessgerät (11) zur Messung des Blutzuckerwertes, wobei die Insulinpumpe mindestens einen Alarmgeber (21) aufweist, und wobei mindestens ein Alarm des Alarmgebers durch ein Signal (18) des Messgerätes (11), das von der Insulinpumpe (1) drahtlos oder drahtgebunden empfangen wird, deaktivierbar ist.

26. Verfahren nach Anspruch 25, wobei der Alarmgeber durch ein Signal des Messgerätes aktiviert wird.

27. Verfahren nach Anspruch 25 oder 26, wobei durch die Insulinpumpe (1) drahtlos oder drahtgebunden ein vom Messgerät (11) empfangbares Signal (19) abgegeben wird, welches einen Alarmgeber des Messgerätes aktiviert und/oder deaktiviert.

28. Verfahren nach einem der Ansprüche 25 bis 27, wobei das Signal ein Signal ist, dass als Information nur die Deaktivierung des Alarms enthält und/oder, dass das Signal mindestens einen vom Messgerätes ermittelten Blutzuckermesswert enthält.

29. Verfahren nach einem der Ansprüche 25 bis 28, wobei ein Alarm deaktiviert wird, wenn ein Signal (18) des Messgerätes enthaltend einen Messwert vorliegt, und die Pumpe (1) derart ausgestaltet ist, dass durch sie das Signal als in einem vorbestimmten Wertebereich liegend erkennbar ist.

30. Verfahren nach einem der Ansprüche 25 bis 29, wobei durch die Pumpe ein deaktivierbarer Alarm als Erinnerungsalarm sofort oder nach einer vorbestimmten Zeit erzeugt wird, nachdem bei der Pumpe (1) ein Ereignis ausgelöst worden ist, insbesondere der Wechsel eines Vorratsbehälters (2) oder die Abgabe eines Mahlzeitenbolus, wobei das Signal des Messgerätes den Alarm vorzugsweise derart deaktiviert, dass die Infusionspumpe auf Grund des Signals des Messgerätes den Erinnerungsalarm deaktiviert, falls das Signal innert einer vorbestimmten, insbesondere einer einstellbaren oder fest programmierten, Zeitspanne vor dem Alarmzeitpunkt vorliegt oder vorgelegen hat.

31. Verfahren nach einem der Ansprüche 25 bis 30, wobei die Pumpe mit einem Alarm ausgestattet ist, der ausgelöst wird, wenn der Pumpenträger während einer vorbestimmten Zeit keine Bedienoperationen an der Pumpe vorgenommen hat, wobei dieser Alarm deaktivierbar ist, wenn das Messgerät (11) mittels eines Signals, oder vorzugsweise mittels mehreren Signalen, angezeigt hat, dass der Körperwert, vorzugsweise der Blutzucker, in dieser Zeit genügend oft gemessen worden ist.

32. Verfahren nach einem der Ansprüche 25 bis 31, wobei der Erinnerungsalarm durch das Signal deaktivierbar ist, indem er zeitlich verschiebbar ist, so dass er, abhängig von dem Signal, anstelle zum vorgesehenen Zeitpunkt, zu einem vorbestimmt späteren Zeitpunkt erfolgt.

33. Verfahren nach einem der Ansprüche 25 bis 32, wobei durch ein Signal des Messgerätes (11) ein Alarm in der Pumpe (1) setzbar ist, falls innert einer vorbestimmten Zeitspanne mehrere Signale (18) vom Messgerät (11) in der Form von Messwerten vorliegen, die von der Pumpe als einen vorgegebenen Messwert, insbesondere Blutzuckermesswert, unterschreitend erkennbar sind

34. Verfahren nach Anspruch 33, wobei die Pumpe (1) so ausgeführt ist, dass bei einer fehlenden Reaktion des Pumpenträgers auf den Alarm innert einer vorgegebenen Zeit eine Funktion der Pumpe selbsttätig ausgelöst wird.

35. Verfahren nach einem der Ansprüche 25 bis 34, wobei die Alarmsetzung bzw. Auslösung bei dem Messgerät (11) mittels des Signals (19) der Pumpe (1) erfolgt, falls ein Bolusbefehl an der Pumpe von dieser ausgeführt worden ist, ohne dass zuvor innert einer vorbestimmten Zeitspanne mit dem Messgerät (11) eine Messung ausgeführt worden ist.

36. Verfahren nach einem der Ansprüche 25 bis 35, wobei Timer, die auf dem Messgerät (11) gesetzt sind über das Signal (18) an die Pumpe (1) übertragen und dort gesetzt werden.

37. Verfahren nach einem der Ansprüche 25 bis 36, wobei die Pumpe (1) und das Gerät (11) so ausgestaltet sind, dass sie Erkennungssignale austauschen, um den Signalempfang von einer nicht beteiligten Einrichtung auszuschliessen.
